# EUROPÄISCHE PATENTSCHRIFT

(11) **EP 0 883 598 B1**
(45) Veröffentlichungstag und Bekanntmachung des Hinweises auf die Patenterteilung: **20.08.2003**
(21) Anmeldenummer: 97903318.0
(22) Anmeldetag: 19.02.1997
(51) Int. Cl.: C07C 215/80, C07C 217/84, C07C 229/44, A61K 7/13

(54) **VERWENDUNG VON AMINOPHENOL-DERIVATEN IN OXIDATIONSHAARFÄRBEMITTEL**
USE OF AMINOPHENOL DERIVATIVES IN OXIDATIVE HAIR DYES
UTILISATION DE DERIVES D'AMINOPHENOL DANS DES COLORANTS D'OXYDATION POUR LES CHEVEUX

(30) Priorität: 29.02.1996 DE 19607751
(43) Veröffentlichungstag der Anmeldung: 16.12.1998
(73) Patentinhaber: Henkel Kommanditgesellschaft auf Aktien, 40589 Düsseldorf-Holthausen (DE)
(72) Erfinder: ROSE, David, D-40723 Hilden (DE); HÖFFKES, Horst, D-40595 Düsseldorf (DE); MEINIGKE, Bernd, D-51399 Burscheid (DE)
(86) Internationale Anmeldenummer: EP9700767
(87) Internationale Veröffentlichungsnummer: WO97031886

(56) Entgegenhaltungen:
- EP-A- 0 011 843
- EP-A- 0 331 144
- WO-A-92/13824
- DE-A- 2 518 393
- DE-A- 4 344 551
- DE-C- 82 445
- FR-A- 1 543 690
- CHEMICAL ABSTRACTS, vol. 51, no. 12, 25.Juni 1957 Columbus, Ohio, US; abstract no. 8799a, G. EKSTRÖM ET AL.: "Diphenylmethane derivatives with antibacterial effect, especially against Myobacterium tuberculosis" XP002030928 & SE 156 812 A 6.November 1956
- J. AMER. CHEM. SOC. (1973), 95(22), 7359-69, XP002030927 HERSHFIELD, ROBERT ET AL: "Lactonization of ring-substituted coumarinic acids. Structural effects on the partitioning of tetrahedral intermediates in esterification"

## Beschreibung

Die Erfindung betrifft die Verwendung von Aminophenol-derivaten Färben von Keratinfasern sowie diese Verbindungen enthaltende Färbemittel.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Gute Oxidationsfarbstoffvorprodukte müssen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme und den Einfluß chemischer Reduktionsmittel, z. B. gegen Dauerwellflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxy-4,5,6-triaminopyrimidin, 2,4-Dihydroxy-5,6-diaminopyrimidin und 2,5,6-Triaminohydroxypyrimidin.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlor-resorcin, 4-Chlor-resorcin, 2-Chlor-6-methyl-3-aminophenol, 2-Amino-4-hydroxypyridin, 2-Methylresorcin und 5-Methylresorcin.

Bezüglich weiterer üblicher Farbstoffkomponenten wird ausdrücklich auf die Colipa-Liste, herausgegeben vom Industrieverband Körperpflege und Waschmittel, Frankfurt, Bezug genommen.

Allein mit einer Entwicklerkomponente oder einer speziellen Kuppler/Entwickler-Kombination gelingt es in der Regel nicht, eine auf dem Haar natürlich wirkende Farbnuance zu erhalten. In der Praxis werden daher üblicherweise Kombinationen verschiedener Entwicklerkomponenten und Kupplerkomponenten eingesetzt. Es besteht daher ständig Bedarf an neuen, verbesserten Farbstoff-Komponenten.

Es war daher die Aufgabe der vorliegenden Erfindung, neue Entwickler-Komponenten zu finden, die die an Oxidationsfarbstoffvorprodukte zu stellenden Anforderungen in besonderem Maße erfüllen.

Es wurde nun gefunden, daß bestimmte, bisher nicht bekannte Aminophenol-Derivate die an Entwicklerkomponenten gestellten Anforderungen in besonders hohem Maße erfüllen. So werden unter Verwendung dieser Entwicklerkomponenten mit den meisten bekannten Kupplerkomponenten brillante Farbnuancen erhalten, die außerordentlich licht- und waschecht sind. Weiterhin zeichnen sich die erzielten Färbungen durch außerordentliche Kaltwellechtheit und Wärmestabilität, sowie durch eine hervorragende Egalisierung aus.

Ein erster Gegenstand der vorliegenden Erfindung ist daher die Verwendung von Aminophenol-Derivaten der allgemeinen Formel (I), 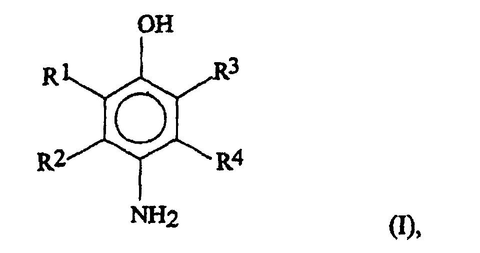 in der eine der beiden Reste R¹ und R² steht fiir Wasserstoff und der andere Rest steht für Wasserstoff, Chlor oder Fluor
und einer der beiden Reste R³ und R⁴ steht fiir
- Wasserstoff
- eine C₁₋₄-Alkylgruppe,
- eine C₁₋₄-Hydroxyalkylgruppe, bevorzugt mit endständiger Hydroxygruppe, oder
- Halogen,
und der andere Rest steht
- für den Fall, daß es sich um den Rest R³ handelt, für eine Gruppe 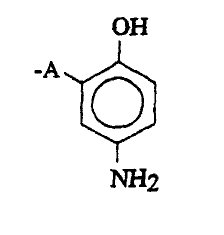
- und für den Fall, daß es sich um den Rest R⁴ handelt, fiir eine Gruppe 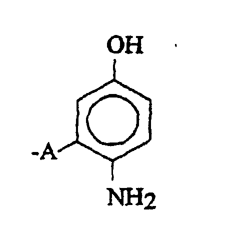 in denen -A- jeweils steht für eine der Gruppen
   -(CH₂)ₓ- mit x = 1 - 4,
   -O-(CH₂)_{y}-O- mit y = 1 - 4,
   -O-(CₙH_{2n-z}(OH)_{z})-O- mit n = 1 - 10 und z = 1 oder wenn n größer oder gleich 3 ist, = 2,
   -O-(C₂H₄-O)ᵤ- mit u = 1 - 4 und
   -O-(C₃H₆-O)ᵥ- mit v = 1 - 4,
   und deren wasserlöslichen Salzen als Entwicklen-Komperente in Oxidadionshaarfärbemitteln.

In der deutschen Offenlegungsschrift DE-A-251 83 93 werden Haarfärbemittel offenbart, die als Farbstoffvorprodukte spezielle über eine direkte Bindung oder ein Sauerstoffatom verbrückte 2-kernige p-Aminophenolderivate enhalten.

In der deutschen Offenlegungsschrift DE-A-434 45 51 sowie der europäischen Offenlegungsschrift EP-A-331 144 werden Haarfärbemittel offenbart, die spezielle 1-kernige p-Aminophenolderivate enthalten.

Diese Verbindungen lassen sich mit bekannten organischen Synthesemethoden herstellen. Bezüglich der Einzelheiten wird ausdrücklich auf die im Beispielteil ausführlich dargestellten Synthesebeispiele verwiesen.

Da es sich bei allen erfindungsgemäßen Substanzen um Amino-Verbindungen handelt, lassen sich aus diesen in üblicher Weise die bekannten Säureadditionssalze herstellen. Alle Aussagen dieser Schrift und demgemäß der beanspruchte Schutzbereich beziehen sich daher sowohl auf die in freier Form vorliegenden Aminophenol-Derivate gemäß Formel (I) als auch auf deren wasserlösliche, physiologisch verträgliche Salze. Beispiele für solche Salze sind die Hydrochloride, die Hydrobromide, die Sulfate, die Phosphate, die Acetate, die Propionate, die Citrate und die Lactate.

Als erfindungsgemäß besonders geeignet haben sich die Aminophenol-Derivate gemäß Formel (I) erwiesen, bei denen sowohl R' als auch R² für Wasserstoff steht.

Ebenfalls erfindungsgemäß bevorzugt sind solche Verbindungen gemäß Formel (I), bei denen einer der beiden Reste R³ oder R⁴ fiir Wasserstoff steht.

Unter den Verbindungen, die Hydroxyalkyl-Diether darstellen, sind schließlich solche bevorzugt, bei denen n für eine Zahl von 1 bis 6 steht und die eine Hydroxygruppe an der aliphatischen Kette aufweisen, d.h. z = 1.

Besonders hervorragend im Sinne der Gesamterfindung geeignete Substanzen sind
- Bis-(5-amino-2-hydroxyphenyl)-methan,
- Bis-[2-(2-hydroxy-5-aminophenoxy)-ethyl]-ether,
- 1,8-Bis[2-hydroxy-5-aminophenoxy]-3,6-dioxaoctan
- 1,3-Bis-(2-hydroxy-5-aminophenoxy)propan und
- 1,3-Bis-(2-hydroxy-5-aminophenoxy)propan-2-ol.

Ein zweiter Gegenstand der vorliegenden Erfindung sind schließlich Oxidationsfärbemittel zum Färben von Keratinfasern enthaltend Kupplerkomponenten und Entwicklerkomponenten in einem wasserhaltigen Träger, die als Entwickler-Komponente eines der vorgenannten Aminophenol-Derivate enthält.

Unter Keratinfasern sind dabei Pelze, Wolle, Federn und insbesondere menschliche Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten, insbesondere in der Farbphotographie, nichts entgegen.

Die erfindungsgemäßen Oxidationsfärbemittel enthalten die erfindungsgemäßen Entwickler-Komponenten und können gewünschtenfalls noch weitere Entwickler-Komponenten sowie Kuppler-Komponenten enthalten. Bezüglich der weiteren Entwickler- und Kupplerkomponenten wird auf die zu Beginn der Beschreibung aufgeführten Substanzen verwiesen, die bevorzugte weitere Farbstoffkomponenten darstellen. Besonders bevorzugte weitere Entwicklerkomponenten sind 2,4,5,6-Tetraaminopyrimidin, 4-Hydroxy-2,5,6-triaminopyrimidin, Dihydroxyaminopyridine, 1-(β-Hydroxyethyl)-2,5-diaminobenzol, p-Phenylendiamin, p-Toluylendiamin, p-Aminophenol, 3-Methyl-4-aminophenol, 2,5-Diaminophenoxyethanol, 2-Hydroxymethyl-p-aminophenol und 2-Aminomethyl-p-aminophenol. 4-Amino-2-(2-hydroxyethoxy)-phenol ist ebenfalls eine Entwickler-Komponente, die bevorzugt mit den erfmdungsgemäßen Entwickler-Komponenten kombiniert werden können. Diese weiteren Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Sulfate, einzusetzen.

Die erfindungsgemäßen Haarfärbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1 : 0,5 bis 1 : 2 enthalten sein können.

In einer bevorzugten Ausführungsform enthalten die erfindungsgemäßen Haarfärbemittel zur weiteren Modifizierung der Farbnuancen neben den Oxidationsfarbstoffvorprodukten zusätzlich übliche direktziehende Farbstoffe, z.B. aus der Gruppe der Nitrophenylendiamine, Nitroaminophenole, Anthrachinone oder Indophenole, wie z.B. die unter den internationalen Bezeichnungen bzw. Handelsnamen HC Yellow 2, HC Yellow 4, Basic Yellow 57, Disperse Orange 3, HC Red 3, HC Red BN, Basic Red 76, HC Blue 2, Nitroblau, Disperse Blue 3, Basic Blue 99, HC Violet 1, Disperse Violet 1, Disperse Violet 4, Disperse Black 9, Basic Brown 16, Pikraminsäure und Rodol 9 R bekannten Verbindungen, in einer Menge von 0,01 bis 20 Gew.-%, bezogen auf das gesamte Oxidationshaarfärbemittel. 4-Amino-2-nitrodiphenylarnin-2'-carbonsäure, 6-Nitro-1,2,3,4-tetrahydrochinoxalin, Rodol 9 R und HC Red BN sind erfindungsgemäß besonders bevorzugte direktziehende Farbstoffe.

Es ist nicht erforderlich, daß die Oxidationsfarbstoffvorprodukte oder die fakultativ enthaltenen direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Zur Herstellung der erfindungsgemäßen Färbemittel werden die Oxidationsfarbstoffvorprodukte in einen geeigneten wasserhaltigen Träger eingearbeitet. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Diund Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäu und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C8-C22-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammoniumglycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Dialkylammoniummethosulfate und Methyl-hydroxyalkyldialkoyloxyalkyl-ammoniummethosulfate.

Ein Beispiel fiir ein als kationisches Tensid einsetzbares quaternäres Zuckerderivat stellt das Handelsprodukt Glucquat® 100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyltrimethylammoniumchlorid/Acrylat-Copolymere und Octylacrylamid/Methylmethacrylat/tert.Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/Ntert.Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsarnengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol. Propylenglykol, Glycerin und Diethylenglykol,
- Farbstoffe zum Einfärben der Zubereitungen,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Weiterhin ist es möglich, die Oxidation mit Hilfe von Enzymen durchzuführen. Dabei können die Enzyme zur Übertragung von Luftsauerstoff auf die Entwicklerkomponente oder zur Verstärkung der Wirkung geringer Mengen vorhandener Oxidationsmittel dienen. Ein Beispiel für ein enzymatisches Verfahren stellt das Vorgehen dar, die Wirkung geringer Mengen (z.B. 1 % und weniger, bezogen auf das gesamte Mittel) Wasserstoffperoxid durch Peroxidasen zu verstärken.

Zweckmäßigerweise wird die Zubereitung des Oxidationsmittels unmittelbar vor dem Haarefärben mit der Zubereitung aus den Oxidationsfarbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die nachfolgenden Beispiele sollen den Erfindungsgegenstand näher erläutern.

### Beispiele

### 1. Synthesebeispiele

### 1.1. Synthese von Bis-(5-amino-2-hydroxyphenyl)-methan

Es wurde folgende Diazonium-Lösung vorbereitet: Zu einer Lösung, bestehend aus 26 g (0,15 Mol) Sulfanilsäure und 75 ml (0,15) 2n Natronlauge wurde bei einer Temperatur von +5 °C 10,35 g (0,15 Mol) Natriumnitrit in 125 ml Wasser zugegeben. Nachdem die Lösung erneut auf +5 °C abgekühlt worden war, wurden während 30 Minuten 131 ml (0,38 Mol) 10 %ige Salzsäure zugetropft. Diese Lösung wurde sodann zu einer eisgekühlten Lösung, bestehend aus 10,0 g (0,05 Mol) Bis-(2-hydroxyphenyl)-methan in 120 ml 10 %iger Natronlauge, zugetropft. Nach beendeter Zugabe wurde noch 1,5 Stunden bei 20 °C nachgerührt.
Danach wurden zu dieser Lösung innerhalb von 2 Minuten 69 g (0,4 Mol) Natriumdithionit zugegeben. Nach 20 Minuten bei 75 °C wurde das Produkt abgesaugt und mit Wasser nachgewaschen.
Die Ausbeute betrug 54 % d.Th.; das Produkt hatte einen Schmelzpunkt von 198 °C.

### 1.2. Bis-[2-(2-hydroxy-5-aminophenoxy)-ethyl]-ether

In einem 600 ml-Becherglas wurden 13,9 g (0,08 Mol) Sulfanilsäure in 41 ml 10%iger Natronlauge gelöst und mit einer Lösung von 5,7 g (0,08 Mol) Natriumnitrit in 30 ml Wasser versetzt. Nach dem Abkühlen der Mischung auf 0 - 5 °C wurden innerhalb von 45 Minuten 72 ml 10%iger Salzsäure zugetropft. Diese kalte Diazonium-Lösung wurde dann in einem Zeitraum von 30 Minuten zu einer Lösung von 11,8 g (0,04 Mol) Bis-([2-(2-hydroxyphenoxy)-ethyl]-ether (hergestellt nach J.Am.Chem.Soc. (1977), 2568) in 66 ml 10 %iger Natronlauge bei 0 - 5 °C zugetropft.. Nach weiterem eineinhalbstündigem Rühren bei 15 °C wurden 38,3 g (0,22 Mol) Natriumdithionit zugegeben. Anschließend wurde 20 Minuten lang auf 75 °C erwärmt. Dabei fiel das Produkt aus. Nach dem Abkühlen wurde das Produkt abgesaugt, mit Wasser nachgewaschen und im Vakuum bei 70 °C getrocknet. Das Produkt hatte einen Schmelzpunkt von 215 °C (unter Zersetzung).

### 1.3. 1,8-Bis[2-hydroxy-5-aminophenoxy]-3,6-dioxaoctan

Die Synthese erfolgte analog Beispiel 1.2., ausgehend von 1,8-Bis-(2-hydroxyphenoxy)-3,6-dioxaoctan. Diese Verbindung wurde nach der Vorschrift aus Ann. (1978), 1594 hergestellt. Das Produkt hatte einen Schmelzpunkt von 144,5 °C (unter Zersetzung).

### 1.4. 1,3-Bis-(2-hydroxy-5-aminophenoxy)propan

Die Synthese erfolgte analog Beispiel 1.2., ausgehend von 1,3-Bis-(2-hydroxyphenoxy)-propan. Diese Verbindung wurde nach der Vorschrift aus J.Org.Chem. 48, 4867 (1983) hergestellt. Das Produkt hatte einen Schmelzpunkt von 150 °C (unter Zersetzung).

### 1.5. 1,3-Bis-(2-hydroxy-5-aminophenoxy)propan-2-ol.

Die Synthese erfolgte analog Beispiel 1.2., ausgehend von 1,3-Bis-(2-hydroxyphenoxy)-propan-2-ol. Diese Verbindung wurde nach der Vorschrift aus J.Chem.Soc., Perkin Trans I (1978),451 hergestellt. Das Produkt hatte einen Schmelzpunkt von 215 °C (unter Zersetzung).

### 2. Ausfärbungen

Es wurde zunächst eine Cremebasis folgender Zusammensetzung hergestellt [alle Angaben sind, soweit nicht anders vermerkt, in g]:

| | |
|---|---|
| Talgfettalkohol | 17,0 |
| Lorol®techn.¹ | 4,0 |
| Texapon®N 28² | 40,0 |
| Dehyton®K³ | 25,0 |
| Eumulgin®B 2⁴ | 1,5 |
| destilliertes Wasser | 12,5 |

| | |
|---|---|
| ¹ C₁₂₋₁₈-Fettalkohol (HENKEL) | |
| ² Natriumlaurylethersulfat (ca. 28 % Aktivsubstanz; CTFA-Bezeichnung: Sodium Laureth Sulfate) (HENKEL) | |
| ³ Fettsäureamid-Derivat mit Betainstruktur der Formel R-CONH(CH₂)₃N⁺(CH₃)₂CH₂COO⁻ (ca. 30 % Aktivsubstanz; CTFA- Bezeichnung Cocoamidopropyl Betaine) (HENKEL) | |
| ⁴ Cetylstearylalkohol mit ca. 20 Mol EO (CTFA-Bezeichnung: Ceteareth-20) (HENKEL) | |

Auf Basis dieser Creme wurde dann folgende Haarfärbecremeemulsion hergestellt:

| | |
|---|---|
| Cremebasis | 50,0 |
| Entwicklerkomponente | 7,5 mmol |
| Kupplerkomponente | 7,5 mmol |
| Na₂SO₃ (Inhibitor) | 1,0 |
| (NH₄)₂SO₄ | 1,0 |
| konz. Ammoniaklösung | ad pH 10 |
| Wasser | ad 100 |

Die Bestandteile wurden der Reihe nach miteinander vermischt. Nach Zugabe der Oxidationsfarbstoffvorprodukte und des Inhibitors wurde zunächst mit konzentrierter Ammoniaklösung der pH-Wert der Emulsion auf 10 eingestellt, dann wurde mit Wasser auf 100 g aufgefüllt.

Die oxidative Entwicklung der Färbung wurde mit 3 %iger Wasserstoffperoxidlösung als Oxidationslösung durchgeführt. Hierzu wurden 100 g der Emulsion mit 50 g Wasserstoffperoxidlösung (3 %ig) versetzt und vermischt.
Die Färbecreme wurde auf ca. 5 cm lange Strähnen standardisierten, zu 90 % ergrauten, aber nicht besonders vorbehandelten Menschenhaars aufgetragen und dort 30 Minuten bei 32 °C belassen. Nach Beendigung des Färbeprozesses wurde das Haar gespült, mit einem üblichen Haarwaschmittel gewaschen und anschließend getrocknet.
Für die Ausfärbungen wurden folgende Kuppler- und Entwickler-Komponenten verwendet:

### Entwickler-Komponenten

- Bis-(5-amino-2-hydroxyphenyl)-methan (El),
- Bis-[2-(2-hydroxy-5-aminophenoxy)-ethyl]-ether (E5),
- 1,8-Bis-(2-hydroxy-5-aminophenoxy)-3,6-dioxaoctan(E6),
- 1,3-Bis-(2-hydroxy-5-aminophenoxy)propan (E7),
- 1,3-Bis-(2-hydroxy-5-aminophenoxy)propan-2-ol (E8) und

### Kuppler-Komponenten

- 2-Methyl-5-aminophenol (K1),
- 1-Naphthol (K2),
- 2-Chlor-6-methyl-3-aminophenol (K3),
- 1,3-Bis-(2,4-diaminophenoxy)propan (K4),
- 2-Methyl-5-(2-hydroxyethylamino)-phenol (K5),
- Resorcin (K6),
- 1,5-Dihydroxynaphthalin (K7),

Es wurden folgende Ausfärbungen gefunden: Nuance des gefärbten Haares

| Entwickler | Kuppler | Kuppler |
|---|---|---|
| E1 | K1 | rotbraun |
| E1 | K2 | violettbraun |
| E1 | K3 | pompejanisch-rot |
| E1 | K4 | dunkelmagenta |
| E1 | K5 | terracotta |
| | | |
| E5 | K3 | lederbraun |
| E5 | K1 | graurot |
| | | |
| E6 | K1 | karamelbraun |
| E6 | K3 | rotbraun |
| | | |
| E7 | K3 | rotbraun |
| E7 | K7 | rehbraun |
| | | |
| E8 | K2 | violettbraun |
| E8 | K7 | violettbraun |

## Patentansprüche

1. Verwendung von Aminophenol-Derivaten der allgemeinen Formel (I), 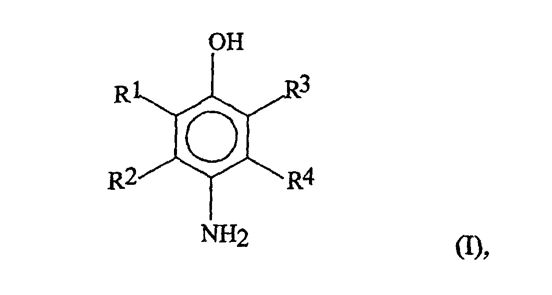 in der einer der beiden Reste R¹ und R² steht für Wasserstoff und der andere Rest steht für Wasserstoff, Chlor oder Fluor,
und einer der beiden Reste R³ und R⁴ steht fiir
- Wasserstoff
- eine C₁₋₄Alkoxygruppe
- eine C₁₋₄-Alkylgruppe,
- eine C₁₋₄-Hydroxyalkylgruppe, bevorzugt mit endständiger Hydroxygruppe, oder
- Halogen,
und der andere Rest steht
für den Fall, daß es sich um den Rest R³ handelt, für eine Gruppe 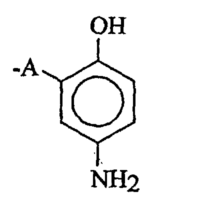
- und für den Fall, daß es sich um den Rest R⁴ handelt, für eine Gruppe 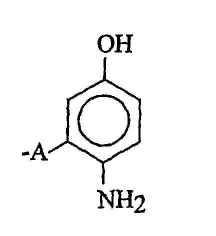
in denen -A- jeweils steht für eine der Gruppen
-(CH₂)ₓ- mit x = 1 - 4,
-O-(CH₂)_{y}-O- mit y = 1 - 4,
-O-(CₙH_{2n-z}(OH)_{z})-O- mit n = 1 - 10 und z = 1 oder, wenn n größer oder gleich 3 ist, = 2,
-O-(C₂H₄-O)ᵤ- mit u = 1 - 4 und
-O-(C₃H₆-O)ᵥ- mit v = 1 - 4,
und deren wasserlöslichen Salzen als Entwickler-Komponente in Oxidationshaarfärbemitteln.

2. Oxidationsfärbemittel zum Färben von Keratinfasem, enthaltend Kupplerkomponenten und Entwicklerkomponenten in einem wasserhaltigen Träger, **dadurch gekennzeichnet, daß** es als Entwickler-Komponente ein Aminophenol- Derivat der allgemeinen Formel (I) oder dessen wasserlösliches Salz enthält.

3. Oxidationsfärbemittel nach Anspruch 2, **dadurch gekennzeichnet, daß** beide Reste R¹ und R² in der allgemeinen Formel (I) für Wasserstoff stehen.

4. Oxidationsfärbemittel nach Anspruch 2 oder 3, **dadurch gekennzeichnet, daß** R³ oder R⁴ in der allgemeinen Formel (I) für Wasserstoff steht.

5. Oxidationsfärbemittel nach einem der Ansprüche 2 bis 4, **dadurch gekennzeichnet, daß** n=1-6, insbesondere 1-3, und z=1 in der allgemeinen Formel (I) ist.

6. Oxidationsfärbemittel nach einem der Ansprüche 2 bis 5, **dadurch gekennzeichnet, daß** es neben den Aminophenol-Derivaten mindestens eine weitere Entwicklerkomponente enthält.

7. Oxidationsfärbemittel nach Anspruch 6, **dadurch gekennzeichnet, daß** die weitere Entwickler-Komponente ausgewählt ist aus 2,4,5,6-Tetraaminopyrimidin, 4- Hydroxy-2,5,6-triaminopyrimidin, 1-(β-Hydroxyethyl)-2,5-diaminobenzol, p-Phenylendiamin, p-Toluylendiamin, p-Aminophenol, 3-Methyl-p-aminophenol und 2-Aminomethyl-p-aminophenol.

8. Oxidationsfärbemittel nach einem der Ansprüche 2 bis 7, **dadurch gekennzeichnet, daß** Entwicklerkomponenten in einer Menge von 0,005 bis 20 Gew.-% vorzugsweise 0,1 bis 5 Gew.-% und Kupplerkomponenten in einer Menge von 0,005 bis 20 Gew.-%, vorzugsweise 0,1 bis 5 Gew.-%, jeweils bezogen auf das gesamte Oxidationsfärbemittel, enthalten sind.

9. Oxidationsfärbemittel nach einem der Ansprüche 2 bis 8, **dadurch gekennzeichnet, daß** weiterhin mindestens ein direktziehender Farbstoff enthalten ist.

## Claims

1. The use of aminophenol derivatives corresponding to general formula (I): 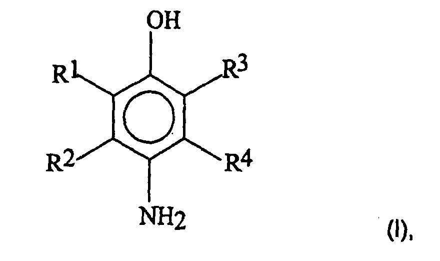 in which
one of the two substituents R¹ and R² is hydrogen and the other substituent is hydrogen, chlorine or fluorine and
one of the two substituents R³ and R⁴ is
- hydrogen,
- a C₁₋₄ alkoxy group,
- a C₁₋₄ alkyl group,
- a C₁₋₄ hydroxyalkyl group, preferably with a terminal hydroxy group, or
- halogen
and the other substituent - where it is the substituent R³ - is the following group: 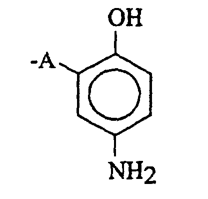 and - where it is the substituent R⁴ - is the following group: 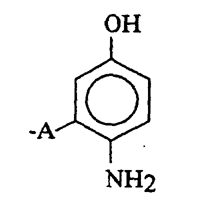 where -A- stands for one of the groups
-(CH₂)ₓ- with x = 1-4,
-O-(CH₂)_{y}-O- with y = 1-4,
-O-(CₙH_{2n-z}(OH)_{z})-O- with n = 1-10 and z = 1 or, where n is 3 or >3, z = 2,
-O-(C₂H₄-O)ᵤ- with u = 1-4 and
-O-(C₃H₆-O)ᵥ- with v = 1-4,
and water-soluble salts thereof as a primary intermediate in oxidation hair colorants.

2. An oxidation colorant for coloring keratin fibers containing primary intermediates and secondary intermediates in a water-containing carrier, **characterized in that** it contains an aminophenol derivative corresponding to general formula (I) or a water-soluble salt thereof as primary intermediate.

3. An oxidation colorant as claimed in claim 2, **characterized in that** both substituents R¹ and R² in general formula (I) are hydrogen.

4. An oxidation colorant as claimed in claim 2 or 3, **characterized in that** R³ or R⁴ in general formula (I) is hydrogen.

5. An oxidation colorant as claimed in any of claims 2 to 4, **characterized in that** n = 1 to 6, more particularly 1 to 3, and z = 1 in general formula (I).

6. An oxidation colorant as claimed in any of claims 2 to 5, **characterized in that**, besides the aminophenol derivatives, it contains at least one other primary intermediate.

7. An oxidation colorant as claimed in claim 6, **characterized in that** the other primary intermediate is selected from 2,4,5,6-tetraaminopyrimidine, 4-hydroxy-2,5,6-triaminopyrimidine, 1-(β-hydroxyethyl)-2,5-diaminobenzene, p-phenylenediamine, p-toluylenediamine, p-aminophenol, 3-methyl-p-aminophenol and 2-aminomethyl-p-aminophenol.

8. An oxidation colorant as claimed in any of claims 2 to 7, **characterized in that** primary intermediates are present in a quantity of 0.005 to 20% by weight and preferably 0.1 to 5% by weight and secondary intermediates are present in a quantity of 0.005 to 20% by weight and preferably 0.1 to 5% by weight, based on the oxidation colorant as a whole.

9. An oxidation colorant as claimed in any of claims 2 to 8, **characterized in that** at least one substantive dye is additionally present.

## Revendications

1. Utilisation de dérivés de l'aminophénol de formule générale (I) 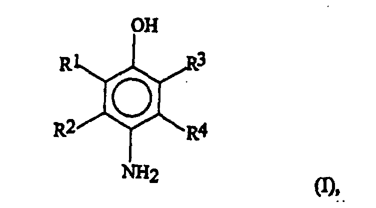 dans laquelle un des deux restes R¹ et R² représente de l'hydrogène et l'autre reste représente de l'hydrogène, du chlore ou du fluor,
et un des deux restes R³ et R⁴ représente
- de l'hydrogène
- un groupe alkoxy en C₁-C₄
- un groupe alkyle en C₁-C₄
- un groupe hydroxyalkyle en C₁ à C₄, de préférence avec un groupe hydroxy terminal ou
- un halogène
et l'autre reste représente dans le cas où s'il s'agit du reste R³, un groupe 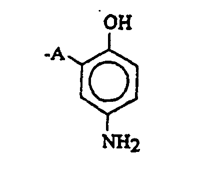 et dans le cas où il s'agit du reste R⁴, un groupe 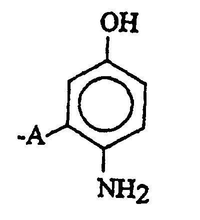 dans lequel
A respectivement représente un des groupes
-(CH₂)ₓ- avec x = 1 - 4,
-O-(CH₂)_{y}-O- avec y = 1 - 4,
-O-(CₙH_{2n-z}(OH)_{z})-O- avec n = 1 - 10 et z = 1 ou quand n est plus grand ou égal à 3 = 2,
-O-(C₂H₄-O)ᵤ- avec u = 1 - 4 et
-O-(C₃H₆-O)ᵥ- avec v = 1 - 4,
et leurs sels hydrosolubles en tant que composant d'agent de développement dans des compositions de teintures pour les cheveux par oxydation.

2. Colorant pour teindre les fibres kératiniques contenant des composants d'agent de couplage et des composants d'agent de développement dans un support contenant de l'eau,
**caractérisé en ce qu'**
il contient comme composant d'agent de développement un dérivé d'aminophénol de formule générale I ou un sel soluble dans l'eau.

3. Colorant par oxydation selon la revendication 2,
**caractérisé en ce que**
les deux restes R¹ et R² représentent dans la formule générale (I) de l'hydrogène.

4. Colorant par oxydation selon la revendication 2 ou la revendication 3,
**caractérisé en ce que**
R³ ou R⁴ représente dans la formule générale (I) de l'hydrogène.

5. Colorant par oxydation selon l'une des revendications 2 à 4,
**caractérisé en ce que**
n est = à 1-6, en particulier 1-3 et z est = 1 dans la formule générale (I).

6. Colorant par oxydation selon l'une des revendications 2 à 5,
**caractérisé en ce qu'**
il contient à côté du dérivé de l'aminophénol au moins un autre composant d'agent de développement.

7. Colorant par oxydation selon la revendication 6,
**caractérisé en ce que**
l'autre composant d'agent de développement est choisi parmi la 2,4,5,6-tétraaminopyrimidine, la 4-hydroxy-2,5,6-triaminopyrimidine, le 1-(β-hydroxyéthyl)-2,5-diaminobenzène, la p-phénylènediamine, la p-toluylènediamine, le p-aminophénol, le 3-méthyl-p-aminophénol et le 2-aminométhyl-p-aminophénol.

8. Colorant par oxydation selon l'une des revendications 2 à 7,
**caractérisé en ce que**
les composants d'agent de développement sont contenus en une quantité de 0,005 à 20 % en poids, de préférence de 0,1 à 5 % en poids et les composants d'agent de couplage en une quantité de 0,005 à 20 % en poids - de préférence de 0,1 à 5 % en poids à chaque fois rapporté à la totalité du colorant par oxydation.

9. Colorant par oxydation selon l'une des revendications 2 à 8,
**caractérisé en ce qu'**
en outre au moins un colorant à action directe est présent.
